Europäisches Patentamt

European Patent Office

Office européen des brevets

(11) Publication number: **0 163 920**
**B1**

(12) **EUROPEAN PATENT SPECIFICATION**

(45) Date of publication of patent specification: **08.08.90**

(21) Application number: **85105114.4**

(22) Date of filing: **26.04.85**

(51) Int. Cl.⁵: **A 61 F 2/30,** A 61 F 2/36,
A 61 B 17/58

(54) **Prosthetic device with predetermined crystal orientation.**

(30) Priority: **21.05.84 US 612083**

(43) Date of publication of application:
**11.12.85 Bulletin 85/50**

(45) Publication of the grant of the patent:
**08.08.90 Bulletin 90/32**

(84) Designated Contracting States:
**SE**

(56) References cited:
EP-A-0 022 724
EP-A-0 145 617
FR-A-2 361 093
US-A-3 877 080

(73) Proprietor: **Pappas, Michael J.**
**61 Gould Place**
**Caldwell, N.J. 07006 (US)**
(73) Proprietor: **Buechel, Frederick F.**
**76 Crest Drive**
**South Orange, N.J. 07079 (US)**

(72) Inventor: **Pappas, Michael J.**
**61 Gould Place**
**Caldwell, N.J. 07006 (US)**
Inventor: **Buechel, Frederick F.**
**76 Crest Drive**
**South Orange, N.J. 07079 (US)**

(74) Representative: **LOUIS, PÖHLAU, LOHRENTZ &**
**SEGETH**
**Ferdinand-Maria-Strasse 6**
**D-8130 Starnberg (DE)**

Courier Press, Leamington Spa, England.

## Description

Structural prosthetic devices such as bone plates, joint replacement stems such as femoral and shoulder stem type prostheses, and intramedullary rods are used to transmit loads between bones or bone segments. Such prostheses are subjected to bending, axial compression, and shear loads. Such bending loads usually produce moments applied about axes perpendicular to a neutral axis representing the locus of points through the centroids of cross-sections of the prostheses taken normal to the neutral axis which is generally along the length of the prosthetic device. Torsion is often also applied about the neutral axis.

Since structural prosthetic devices are generally made of metal which is relatively rigid compared to bone, these devices frequently transfer load between bones or bony segments in a manner which greatly reduces loading on the bone in certain regions thereby inhibiting proper healing of the bone. It is a known property of bone that bone must be subjected to loading or disuse-atrophy will occur. Such protection of the bone, typically called stress shielding, frequently results in disuse-atrophy of certain regions of the bone providing an unhealthy situation for fixation of joint replacement prostheses or fracture fixation prostheses. This is particularly true where metallic fracture fixation prostheses must be removed, as is now recommended by some experts in the field, to avoid release of metal corrosion products into the body. Such removal will eliminate the structural contribution of the metallic fixation prostheses leaving only weakened bone to resist loading and such removal may produce fracture of the bone unless healthy healing has occurred. It is often, therefore, desirable to limit the stiffness of structural prostheses against certain loading modes so that at least after initial healing, preferably, as much of the load as possible is transmitted between bone or bony segments by the bone or bony segments themselves and that the prostheses simply act to align the segments and connect the prostheses to bone.

Further, where fixation plates are used to align fractured or resected bone segments the stiffness of the plate can inhibit bone growth between bone segments. Thus, bending flexibility is desired in order to increase the load transmitted between segments thereby stimulating bone growth.

Several investigators have been experimenting with plastic and composite plates in order to minimize the excessive stiffness of structural prostheses. While the development of such composite or plastic structural prostheses has much potential, the body is a hostile environment and plastics and composites frequently are severely affected by aging and exposure to the environment within the body. Further, there is limited knowledge about how plastics and composites capable of supporting the needed loads would behave in the body over long periods of time and what the effects of the corrosion products resulting from exposure of these materials to body fluids under the action of stresses would be. The biocompatibility or the ability of the body to tolerate such plastics is not well understood. Conversely, metals (in particular cobalt chromium and titanium alloys) have been used in the body for several decades and they are found to be fatigue resistant, relatively corrosion resistant, and well tolerated by the body. Thus, it is advantageous to use these materials at least until such time as plastics and composites are proven to be effective in such use.

A principal advantage of titanium and its alloys for use in femoral stems arises from the fact that these alloys are substantially less rigid than cobalt-chromium or other alloys used in this application. This increased flexibility results in lower stem loading, reducing stem stresses, and higher loading of the bone, reducing disuse-atrophy. Unfortunately, the notch sensitivity of surgical titanium alloy and the attendant drastic reduction in fatigue strength resulting from the use of a metallic porous surface on this material appears to limit the usefulness of porous coated titanium alloys. Thus, titanium alloys may not be well adapted for the exploitation of the substantial benefits available from the use of such coatings for both cement and biological fixation, but is a material of choice where such coatings are not used. On the other hand, cobalt chromium alloys do not appear to be notch sensitive, and seem well adapted for such exploitation.

The stiffness of structural prosthetic devices or fixation prostheses can, of course, be affected by the design of the prosthesis itself. However, strength and geometric requirements (for example the need for a femoral stem to fit adequately into a femoral intramedullary canal) often limit the ability to provide as flexible a structural prosthesis as one would like. The use of more flexible metals such as titanium is also advantageous in this application since they can produce additional prosthesis flexibility. However, even with the use of titanium there are instances where a still more flexible metallic material would be preferred. Furthermore, many orthopaedic surgeons prefer to use cobalt chromium alloys since there is much more experience with the use of these alloys in the body than there is with titanium. Thus, there is a further need in the structural prosthetic device art for biocompatible materials, in particular metals, of increased flexibility.

In some instances such as the use of bone plates for fracture fixation, increased bending and axial flexibility are desirable but increased torsional flexibility is undesirable since torsional stiffness helps resist movement between bone segments thereby stabilizing the fracture and thus promoting healing.

It has been discovered that single crystal materials developed for use as turbine blades in jet engines offer the potential for providing such increased flexibility in preferred directions while simultaneously providing increased stiffness in other preferred directions. The manufacture of parts using these alloys is described in a chapter entitled "The Development of Single Crystal Superalloy Turbine Blades" by M. Gell et al. in a book entitled "Superalloys 1980" which is a publication of the Proceedings of the Fourth International Symposium on Superalloys published in 1980; the article is found on pages 205—215 of the book which is published by The American Society for Metals. Crystals are anisotropic with respect to material properties. Stiffness and strength depend on direction. Cubic crystals, for example, have substantially lower stiffness in the <001> direction. Metals conventionally used for structural or fixation prostheses are polycrystalline in nature and generally have equiaxed grains. Such materials exhibit isotropic properties since the grain and crystal orientations are random and the stiffness is thus intermediate in value between the minimum and maximum crystal stiffness values.

Analogous technology to the single crystal materials is the development of directionally solidified materials or polycrystalline columnar grain structure. Such directionally solidified alloys are discussed in an article by Francis L. VerSnyder and N. E. Shank entitled "The Development of Polymer Grain in Single Crystal High Temperature Metals Through Directional Solidification" appearing in Material Science and Engineering, Vol. 6, No. 4, 1970, pp. 213—247; such alloys are also discussed in US-Patent No. 3.677.835.

From FR—A—23 61 093 it is known to provide an endo-prosthesis the structure of which is adapted to the anisotropy of the bone material. For this purpose there is suggested that a material which is physiologically compatible with the bone material be combined with reinforcement means which provide the required structural rigidity of the endo-prosthesis, whereby the reinforcement means preferably is made of a fibre material, the bending rigidity of which is aligned with the anisotropy of the bone material, that is the greater stiffness thereof is aligned with the maximum stresses occurring in the bone. Besides, there is mentioned the use of a single crystal material for such reinforcement means, which, in accordance with the general teaching of this prior art, is adapted to the anisotropy of the bone material in such a way that its maximum modulus of elasticity is aligned with the maximum stresses occurring in the bone.

It is the basic object of the instant invention to provide an endo-prosthesis including a metallic portion which is more closely adapted to the load transferring structure of the bone material so as to facilitate bone or bone segment healing with an attendant reduction in bone disuse-atrophy.

This object is attained by the structure claimed in claims 1 and 3.

The invention generally relates to use of metallic structural prosthetic devices in which the crystalline structure of the metal is organized and oriented such that the flexibility and/or stiffness of the material is greater in certain preferred directions compared to conventional polycrystalline materials commonly used in the prosthetic art; appropriately used, the flexibility increases the rate of bone regeneration thereby facilitating bone or bone segment healing with an attendant reduction in bone disuse-atrophy, and appropriately used, the stiffness provides increased bone segment stabilization thereby facilitating bone segment healing.

Description of the drawings

Fig. 1 is a diagrammatic side view, generally in cross-section, of a stem-type femoral prosthesis embodying the present invention and shown fixtured in the proximal end of a femur;

Fig. 1a is a diagrammatical illustration of cubic crystal structure of a single crystal material with a crystal direction having a preferred orientation in the (001) crystal direction;

Fig. 2 is a diagrammatic illustration of polycrystalline columnar grain structure consisting of grain columns having a crystal direction with a preferred orientation in the (001) direction;

Figs. 3 and 4 are, respectively, side and front elevational views of a fracture fixation or bone plate embodying the present invention and shown fixtured to bone or bony segments;

Fig. 5 is a diagrammatical illustration of an intramedullary fixation rod inserted into bone or bony segments for maintaining the segments in alignment and for facilitating bone healing;

Fig. 6 is a cross-sectional view taken generally along the cross-sectional line 6—6 of Fig. 5;

Fig. 7 is a diagrammatical side view of a stem-type humeral prosthesis embodying the present invention and shown fixtured in the proximal end of a humerus;

Figs. 7a and 7b are views similar to Fig. 1a illustrating preferred crystal direction orientation; and

Fig. 8 is a diagrammatic side view, generally in cross-section, of a press-fit stem-type femoral prosthesis embodying the present invention and shown fixtured in the proximal end of a femur.

Description of the preferred embodiments

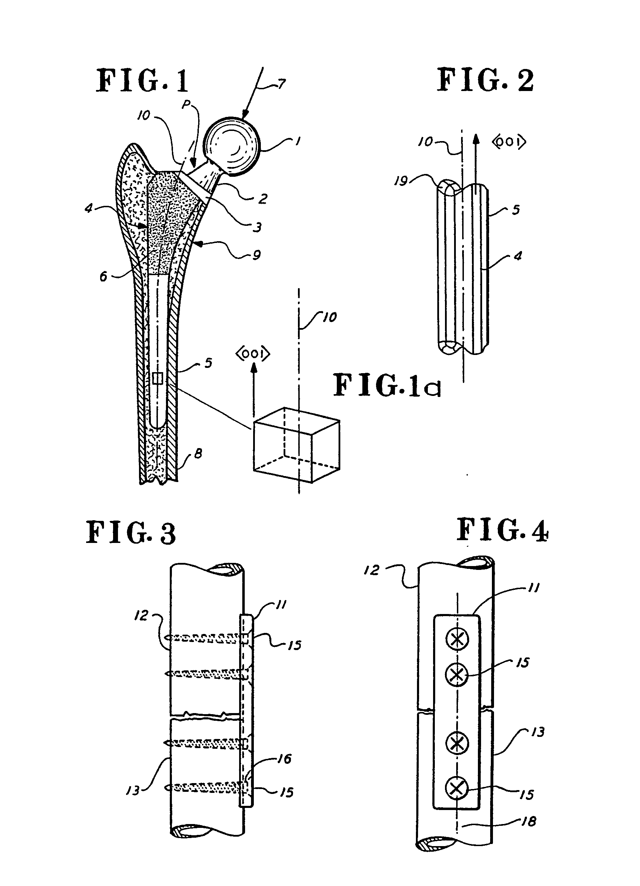

Five embodiments of the invention are shown in the Figs. Fig. 1 shows an embodiment of a stem-type femoral prosthesis P having a head 1, a neck 2, a collar 3 and a stem 4. The stem 4 is fixtured in the intramedullary cavity of a femur 5 along the outside surface of the stem by either the use of acrylic cement or by direct bone-ingrowth or biological fixation into a porous coating 6 on stem 4. The stem 4 transmits an applied load 7 from the acetabulum (not shown) to the shaft 8 of femur 5. Load 7 results in the axial compression shear and bending of the prosthesis P and femur 5 to which it is attached. Stem 4 and femur 5 act as a composite structure both sharing these applied loads. The proportion of loading shared by each structure is dependent on the relative stiffnesses of the structures with the stiffer structure generally carrying the greater portion of the applied shared load. It is desirable to prevent disuse-atrophy of the femur 5 by allowing the femur rather than the stem 4 to take most of the load. However, in conventional stem materials, particularly relatively rigid alloys such as cobalt chromium alloy, the stem carries the bulk of the applied shared load in the region of the proximal femur as indicated generally by region 9 thus often producing disuse-atrophy and resorption of the femur in this region.

In the present invention, the femoral stem-type prosthesis P and in particular the stem 4 is made of a single crystal material or metal of cubic crystal structure wherein the <001> crystal direction has an orientation generally along or parallel to the direction of a neutral axis 10. Alternately, the stem 4 could be cast with a polycrystalline columnar grain structure as shown in Fig. 2 consisting of grain columns 19 with a crystal orientation where the <001> direction is parallel to the stem neutral axis 10. Under the loading conditions shown in Fig. 1, the stem 4 and femur 5 acts as a beam-column. In such a loading condition, the principal strain directions in the stem are essentially parallel to the neutral axis. Thus, for example, where a stem is formed of a single crystal with the <001> crystal direction substantially parallel to the neutral axis 10, such stem is significantly less stiff under the action of normally applied loads than an equiaxed conventional stem of the same material. With respect to bending and compression, a single crystal material stem of the construction described (e.g., single crystal cobalt chromium alloy) would possess for practical purposes a tensile modulus approximately equal to $12,45 \times 10^6$ N/cm$^2$ as compared to a tensile modulus of about $20,7 \times 10^6$ N/cm$^2$ for equiaxed grain material. (The application of single crystal technology to the manufacture of titanium stems in order to increase flexibility may also be utilized to improve the flexibility of titanium and other metallic stems.) As a result of this increased flexibility, the bone in region 9 (Fig. 1) will experience greater loading thereby reducing disuse-atrophy and resorption and thereby promoting bone healing and providing a healthier condition for maintenance of satisfactory bone stock in order to maintain fixation of the structural prosthesis, e.g., stem 4.

A second embodiment of the invention is shown in Figs. 3 and 4 wherein the embodiment is a fracture fixation or bone plate 11. Such a plate is used to hold together bone or bony segments 12 and 13 so that these segments may be aligned during the bone healing process. The bone plate 11 is generally held to the bony segments 12 and 13 by the use of four or more screws 15 screwed into the bony segments through holes 16 in the plate. This plate is subjected to loading conditions similar to that of the femoral stem-type prosthesis discussed earlier, namely this loading includes axial compression, bending and torsion. Here again, if the plate 11 is made of single crystal material so that the <001> crystal orientation is directed along a neutral axis 18, the flexibility of the plate 11 with respect to the axial compression which is generally directed parallel to the neutral axis 18, bending moments which are generally acting about an axis perpendicular to the neutral axis and torsion which supplies the twisting moment generally parallel to the neutral axis, then the bone plate 11 will be more flexible in response to the bending and axial compression action on this shared applied loading than would be a bone plate made of equiaxed polycrystalline grains of a similar material as is the conventional practice. The bone plate 11, as the stem 4 of Fig. 1, could be cast with the columnar grain structure and crystal orientation of Fig. 2. It should be noted that the plate 11 is also stiffer in torsion than an equiaxed polycrystalline material since the crystal is stiffer in directions <111> and <101> which are associated with shear deformation. This increased stiffening effect is greater for a single crystal than for directionally solidified columnar grains.

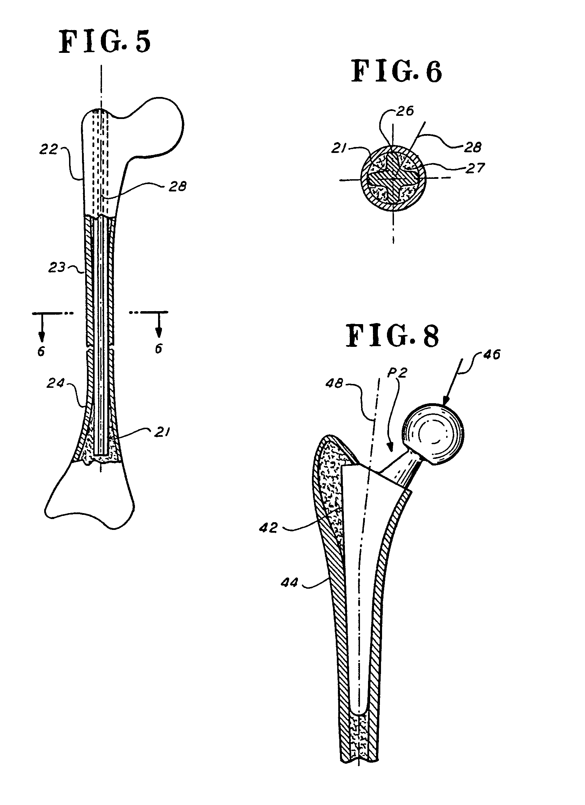

The third embodiment is shown in Figs. 5 and 6. This embodiment is an intramedullary fixation rod 21 shown inserted into the intramedullary cavity of a bone such as the femur 22 in order to hold bony segments 23 and 24 in alignment during the bone healing process. Intramedullary rod 21 is typically fluted on its outside surface as shown in Fig. 6 having projections 26 and depressions 27 providing twisting resistance between the rod and bone about the neutral axis 28 of the rod. This rod is subjected to forces similar to those described in regard to the bone plate 11 (Figs 3 and 4) except that in the case of the rod 21 smoothness of its outer surface allows only minor transfer of compressive load along the rod and thus loading is dominated primarily by bending and torsion. In accordance with the teachings of the present invention, if the rod 21 is made of material, e.g. single crystal or columnar grain structure, such that crystalline orientation of the <001> direction of the material is made parallel to the neutral axis 28 of the rod 21, then the rod will be made more flexible with respect to bending and stiffer in torsion than a similar rod of the same material but of conventional equiaxed polycrystalline orientation material.

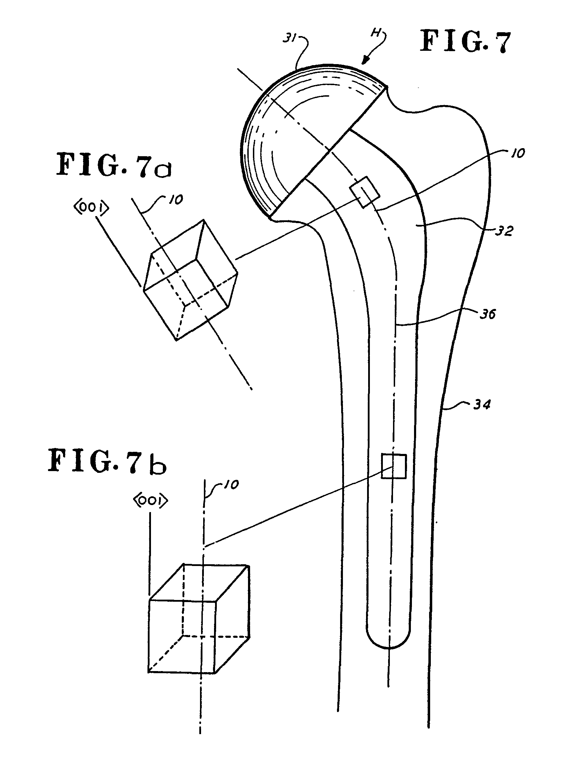

The fourth shown invention embodiment is a stem-type humeral prosthesis H of Fig. 7 having a head 31 and a stem 32 fixtured in the proximal end of a humerus 34. The stem 32 has a neutral axis 36 and the stem 32, in accordance with the teachings of the present invention and as set forth above with regard to the femoral stem-type prosthesis P of Fig. 1 may be made of single crystal or polycrystalline columnar grain structure having a crystal orientation where the <001> direction is parallel to the neutral axis 36 as illustrated by the cubes of Figs. 7a and 7b. Such crystal direction orientation provides the same benefits with regard to reduction in bone disuse-atrophy and facilitation of bone healing as taught above with regard to the femoral prosthesis P of Fig. 1.

The fifth invention embodiment is a press-fit stem-type prosthesis such as the press-fit stem-type femoral prosthesis P2 illustrated in Fig. 8. The need for increased stem flexibility is particularly great for press-fit stem-type prostheses. The stem 42 of the prosthesis P2, as known to those skilled in the art, is made relatively thick as compared to conventional stems such as the stem 4 of the prosthesis P of Fig. 1 in order to fit tightly within the intramedullary canal of the proximal femur 44. With such a press-fit type stem, the shared load 46 is transmitted primarily by a wedging type contact similar to a tapered plug wedged into a tapered hole. The relatively large thickness or cross-sectional size of such stems results in a much stiffer stem than the noted conventional stems. Thus, press-fit type stems may increase stress shielding and its attendant disuse-atrophy. Thus, it will be understood that in accordance with the further teachings of this invention, a thicker stem 42 of a single crystal or of directionally solidified material having the crystal direction orientations illustrated in Figs. 1a and 2 with respect to the neutral axis 48 (Fig. 8) will be provided with increased flexibility and attendant reduction in bone disuse-atrophy and enhanced bone healing, as taught above. Such increased flexibility is of particularly great value with such thicker press-fit type stems.

It will be understood that the present invention is equally applicable to other stem-type structural prostheses such as, for example, finger, toe, wrist and tibial prostheses.

## Claims

1. A prosthetic device for fixation to bone needing healing, the device comprising a predetermined substantially elongated portion (4, 11, 21, 32, 42) which is determined to be directly fixed to said bone in such a way that said elongated portion and said bone are subject to respective portions of a load applied to the prosthetic device upon fixation thereof, said predetermined substantially elongated portion having a neutral axis (10, 18, 28, 36, 48) and including a single crystal material, characterized in that said predetermined substantially elongated portion (4, 11, 21, 32, 42) consists entirely of a single crystal formed from molten metal along said neutral axis (10, 18, 28, 36, 48) and having a predetermined crystal direction oriented parallel with respect to said neutral axis to provide said predetermined substantially elongated portion with an increase in flexibility in that direction of said neutral axis, upon said fixation said increase in flexibility causing a decrease in the portion of said load carried by said predetermined substantially elongated portion and an increase in the portion of said load carried by said bone which facilitates healing of said bone by reducing bone disuse atrophy.

2. A prosthetic device according to claim 1 wherein said predetermined substantially elongated portion comprises a fracture fixation bone plate (11) for fixation to bone segments (12, 13), and wherein at least one further predetermined crystal direction provides said bone plate (11) with increased stiffness for resisting movement between said bone segments in a direction different from the direction of said neutral axis (18).

3. A prosthetic device for fixation to bone needing healing, the device comprising a predetermined substantially elongated portion (4, 11, 21, 32, 42) which is determined to be directly fixed to said bone in such a way that said elongated portion and said bone are subject to respective portions of a load applied to the prosthetic device upon fixation thereof, said predetermined substantially elongated portion having a neutral axis (10, 18, 28, 36, 48) characterized in that said predetermined substantially elongated portion (4, 11, 21, 32, 42) consists entirely of polycrystalline columnar grain metallic structure consisting of grain columns (19) formed from molten metal along said neutral axis (10, 18, 28, 36, 48) and having a predetermined crystal direction oriented parallel with respect to said neutral axis to provide said predetermined substantially elongated portion with an increase in flexibility in that direction of said neutral axis, upon said fixation said increase in flexibility causing a decrease in the portion of said load carried by said predetermined substantially elongated portion and an increase in the portion of said load carried by said bone which facilitates healing of said bone by reducing bone disuse atrophy.

4. A prosthetic device according to claim 1 or 3, wherein said single crystal or polycrystalline columnar grain structure comprise cubic crystal structure and wherein said predetermined crystal direction is in the (001) direction.

5. A prosthetic device according to claim 1 or 3 wherein said prosthetic device is a stem-type femoral or humeral prosthesis including a stem (4, 42; 32) and wherein said predetermined substantial elongated portion comprises said stem.

6. A prosthetic device according to claim 1 or 3 wherein said prosthetic device is an intramedullary fixation rod (21) and wherein said predetermined substantially elongated portion comprises the entirety of said rod.

7. A prosthetic device according to any of claims 1 to 5 wherein said prosthetic device is a press-fit stem-type prothesis including a stem and wherein said predetermined substantially elongated portion comprises said stem.

8. A prosthetic device according to claim 7 wherein said press-fit stem-type prosthesis is a press-fit stem-type femoral prosthesis.

9. A prosthetic device according to claim 1 or 3 wherein said predetermined crystal direction is in the (001) direction.

10. A prosthetic device according to claim 2 wherein said further predetermined crystal direction is in the (101) or (111) direction.

## Patentansprüche

1. Prothese zur Fixierung an einem Heilung erfordernden Knochen, mit einem bestimmten, im wesentlichen länglichen Abschnitt (4, 11, 21, 32, 42), der bestimmt ist zu einer unmittelbaren Fixierung an dem Knochen in der Weise, daß der längliche Abschnitt und der Knochen jeweils anteilig einer nach der Fixierung der Prothese auf diese aufgebrachten Belastung unterliegen, wobei der im wesentlichen längliche Abschnitt eine neutrale Achse (10, 18, 28, 36, 48) hat und ein Einkristallmaterial aufweist, dadurch gekennzeichnet, daß der bestimmte, im wesentlichen längliche Abschnitt (4, 11, 21, 32, 42) vollständig aus einem Einkristall besteht, der sich längs der neutralen Achse (10, 18, 28, 36, 48) aus einer Metallschmelze gebildet hat und eine vorbestimmte Kristallrichtung mit zu der neutralen Achse paralleler Ausrichtung hat, um dem länglichen Abschnitt eine erhöhte Nachgiebigkeit in Richtung der neutralen Achse zu geben, wobei nach der Fixierung diese Zunahme an Nachgiebigkeit eine Verringerung des durch den länglichen Abschnitt aufzunehmenden Belastungsanteils und eine Erhöhung des durch den Knochen aufzunehmenden Belastungsanteils bewirkt, was die Heilung des Knochens durch eine Verminderung der Knochen-Atrophie infolge Nichtgebrauches erleichtert.

2. Prothese nach Anspruch 1, dadurch gekennzeichnet, daß der im wesentlichen längliche Abschnitt eine Bruchfixierungs-Knochenplatte (11) zur Fixierung an Knochenteilen (12, 13) aufweist und daß zumindest eine weitere vorbestimmte Kristallrichtung dieser Knochenplatte (11) eine erhöhte Steifigkeit vermittelt, um einer Bewegung zwischen den Knochenteilen in einer von der Richtung der neutralen Achse (18) abweichenden Richtung entgegenzuwirken.

3. Prothese zur Fixierung an einem Heilung erfordernden Knochen, mit einem bestimmten, im wesentlichen länglichen Abschnitt (4, 11, 21, 32, 42), der bestimmt ist zu einer unmittelbaren Fixierung an dem Knochen in der Weise, daß der längliche Abschnitt und der Knochen jeweils anteilig einer nach der Fixierung der Prothese auf diese aufgebrachten Belastung unterliegen, wobei der im wesentlichen längliche Abschnitt eine neutrale Achse (10, 18, 28, 36, 48) hat, dadurch gekennzeichnet, daß der bestimmte, im wesentlichen längliche Abschnitt (4, 11, 21, 32, 42) vollständig aus einer polykristallinen säulenartigen Metallkorn-Struktur besteht, die aus längs der neutralen Achse (10, 18, 28, 36, 48) aus Metallschmelze gebildeten Kornsäulen (19) aufgebaut ist und eine vorbestimmte Kristallrichtung mit zu der neutralen Achse paralleler Ausrichtung hat, um dem länglichen Abschnitt eine erhöhte Nachgiebigkeit in Richtung der neutralen Achse au geben, wobei nach der Fixierung diese Zunahme an Nachgiebigkeit eine Verringerung des durch den länglichen Abschnitt aufzunehmenden Belastungsanteils und eine Erhöhung des durch den Knochen aufzunehmenden Belastungsanteils bewirkt, was die Heilung des Knochens durch eine Verminderung der Knochen-Atrophie infolge Nichtgebrauches erleichtert.

4. Prothese nach Anspruch 1 oder 3, dadurch gekennzeichnet, daß der Einkristall bzw. die polykristalline säulenartige Kornstruktur eine kubische Kristallstruktur beinhaltet, und daß die vorbestimmte Kristallrichtung die (001)-Richtung ist.

5. Prothese nach Anspruch 1 oder 3, dadurch gekennzeichnet, daß sie eine schaftartige Femur- oder Humerus-Prothese mit einem Schaft (4, 42; 32) ist und daß der bestimmte, im wesentlichen längliche Abschnitt diesen Schaft aufweist.

6. Prothese nach Anspruch 1 oder 3, dadurch gekennzeichnet, daß sie ein intramedullärer Fixierstab (21) ist und daß der bestimmte, im wesentlichen längliche Abschnitt diesen ganzen Stab bildet.

7. Prothese nach einem der Ansprüche 1 bis 5, dadurch gekennzeichnet, daß sie eine durch Druck einzupassende schaftartige Prothese mit einem Schaft ist und daß der bestimmte, im wesentlichen längliche Abschnitt diesen Schaft bildet.

8. Prothese nach Anspruch 7, dadurch gekennzeichnet, daß die durch Druck einzupassende schaftartige Prothese eine durch Druck einzupassende schaftartige Femur-Prothese ist.

9. Prothese nach Anspruch 1 oder 3, dadurch gekennzeichnet, daß die vorbestimmte Kristallrichtung die (001)-Richtung ist.

10. Prothese nach Anspruch 2, dadurch gekennzeichnet daß die weitere vorbestimmte Kristallrichtung die (101)- oder (111)-Richtung ist.

**Revendications**

1. Dispositif de prothèse pour la fixation à un os nécessitant des soins, comprenant une partie prédéterminée, essentiellement allongée (4, 11, 21, 32, 42) qui est prévue pour être fixée directement à l'os susdit de manière telle que cette partie allongée et l'os soient soumis à des portions correspondantes d'une charge appliquée au dispositif de prothèse à la fixation de celui-ci, cette partie prédéterminée, essentiellement allongée présentant un axe neutre (10, 18, 28, 36, 48) et comprenant une matière monocristalline, caractérisé en ce que cette partie prédéterminée, essentiellement allongée (4, 11, 21, 32, 42) consiste totalement en un monocristal formé au départ d'un métal en fusion, le long de l'axe neutre susdit (10, 18, 28, 36, 48) et présentant une direction cristalline prédéterminée orientée parallèlement par rapport à l'axe neutre susdit pour donner à cette partie prédéterminée, essentiellement allongée, une augmentation de flexibilité dans la direction de l'axe neutre susdit, cette augmentation de flexibilité, à la fixation susdite, provoquant une diminution de la partie de la charge qui est supportée par cette partie prédéterminée, essentiellement allongée, et une augmentation de la partie de cette charge supportée par l'os, ce qui facilite la guérison de celui-ci en réduisant l'atrophie due à un manque d'utilisation de l'os.

2. Dispositif de prothèse suivant la revendication 1, dans lequel la partie prédéterminée susdite, essentiellement allongée, comprend une plaque pour os (11) destinée à une fixation à des segments d'os (12, 13), et dans lequel au moins une autre direction cristalline prédéterminée apporte à cette plaque pour os (11) und rigidité accrue en vue de résister au mouvement entre les segments d'os susdits dans une direction différente de la direction de l'axe neutre précité (18).

3. Dispositif de prothèse destiné à la fixation d'un os nécessitant des soins, ce dispositif comprenant une partie prédéterminée, essentiellement allongée (4, 11, 21, 32, 42) qui est conçue pour être fixée directement à l'os de manière telle que cette partie allongée et cet os soient soumis à des portions correspondantes d'une charge appliquée au dispositif de prothèse à la fixation de celui-ci, cette partie prédéterminée, essentiellement allongée, présentant un axe neutre (10, 18, 28, 36, 48), caractérisé en ce que cette partie prédéterminée, essentiellement allongée (4, 11, 21, 32, 42) consiste totalement en une structure métallique polycristalline, à grains en colonne, constituée de colonnes de grains (19) formés d'un métal fondu, le long de l'axe neutre susdit (10, 18, 28, 36, 48) et présentant une direction cristalline prédéterminée orientée parallèlement à l'axe neutre précité pour donner à cette partie prédéterminée, essentiellement allongée, une augmentation de flexibilité dans la direction de cet axe neutre, cette augmentation de flexibilité, à la fixation susdite, provoquant une diminution de la partie de la charge supportée par la partie prédéterminée, essentiellement allongée, et une augmentation de la partie de la charge supportée par l'os, ce qui facilite la guérison de celui-ci en réduisant l'atrophie due à un manque d'utilisation de cet os.

4. Dispositif de prothèse suivant la revendication 1 ou la revendication 3, dans lequel la structure monocristalline susdite ou la structure polycristalline à grains en colonne consiste en une structure cristalline cubique, la direction prédéterminée des cristaux étant la direction (001).

5. Dispositif de prothèse suivant la revendication 1 ou la revendication 3, caractérisé en ce qu'il consiste en une prothèse fémorale ou humérale du type à tige, comprenant une tige (4, 42; 32), la partie prédéterminée, essentiellement allongée susdite, consistant en cette tige.

6. Dispositif de prothèse suivant la revendication 1 ou la revendication 3, qui est constitué par une tige de fixation intramédulaire (21), la partie prédéterminée, essentiellement allongée susdite étant constituée par la totalité de cette tige.

7. Dispositif de prothèse suivant l'une quelconque des revendications 1 à 5, caractérisé en ce qu'il s'agit d'une prothèse du type à tige, à adaptation par pression, comprenant une tige et dans lequel la partie prédéterminée essentiellement allongée consiste en cette tige.

8. Dispositif de prothèse suivant la revendication 7, caractérisé en ce que la prothèse du type à tige, à montage par pression, est une prothèse fémorale du type à tige, à montage par pression.

9. Dispositif de prothèse suivant la revendication 1 ou la revendication 3, caractérisé en ce que la direction cristalline prédéterminée est la direction (001).

10. Dispositif de prothèse suivant la revendication 2, dans lequel la direction cristalline prédéterminée supplémentaire susdite est la direction (101) ou (111).

FIG. 1

FIG. 2

FIG. 1a

FIG. 3

FIG. 4

# FIG. 5

# FIG. 6

# FIG. 8

FIG. 7

FIG. 7a

FIG. 7b